# EUROPEAN PATENT APPLICATION

(11) **EP 1 000 925 A1**
(43) Date of publication of application: **17.05.2000**
(21) Application number: 98924653.3
(22) Date of filing: 15.06.1998
(51) Int. Cl.: C07C 217/32, C07C 271/44, A61K 31/135, A61K 31/27

(54) **TRIPHENYLMETHANE DERIVATIVES AND USE THEREOF**

(30) Priority: 17.06.1997 JP 15990597
(71) Applicant: Sumitomo Chemical Company, Limited, Chuo-ku Osaka 541-8550 (JP)
(72) Inventor: KANEKO, Hideo, Kobe-shi Hyogo 658-0046 (JP); TOMIGAHARA, Yoshitaka, Osaka 561-0802 (JP); OOE, Norihisa, Osaka 530-0041 (JP); MATSUNAGA, Haruyuki, Osaka-shi Osaka 552-0004 (JP); NAKATSUKA, Iwao, Hyogo 651-1233 (JP)
(74) Representative: VOSSIUS & PARTNER
(86) International application number: JP9802659
(87) International publication number: WO9857922

(57) **Abstract**

Triphenylmethane derivatives represented by the general formula: 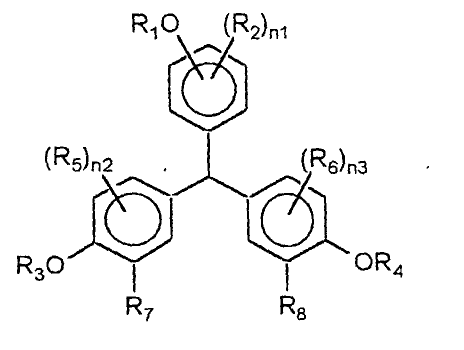 as well as pharmaceutical compositions comprising the same as an active ingredient are provided.

## Description

### Field of the Invention

The present invention relates to triphenylmethane derivatives and salts thereof as well as pharmaceutical compositions comprising the same.

### Prior Art

Estrogen receptors exist on the target cells for estrogens in tissues such as uterus, vagina, oviduct, liver, breast carcinoma, and bone, and when bound to estrogens, they induce protein syntheses which depend on particular genes. It is known that various diseases are caused by abnormal activation of such estrogens and receptors, and by the consequent promotion of the protein synthesis. Thus, there have been various attempts to use, as clinical remedies for such diseases, substances which exhibit antiestrogenic activities by acting on estrogen receptors. As an example of such attempts, it has been known to use an antiestrogen, tamoxifen, as a remedy for breast cancer. In such treatments with antiestrogens, however, involvement of various undesired symptoms are also observed, rendering such treatments difficult to use.

Recently, the β-isoform of estrogen receptor distinct from the conventional α-isoform has been discovered (*Proc. Natl. Acad. Sci. USA,* vol. 93, 5925-5930 (1996)). This finding has suggested that respective receptors may have different roles. It is expected based on such findings that once an antiestrogen which has different affinities for the α- and β-isoforms and thereby selectively acts on one of the two isoforms could be developed, such antiestrogen may alleviate undesired effects which may be caused by the action of the antiestrogen on the other isoform. It is therefore earnestly desired to identify such substances which exhibits antiestrogenic effects on estrogen receptors in an isoform-dependent manner.

### Disclosure of the Invention

In such circumstances, the present inventors have found after their extensive efforts that certain triphenylmethane derivatives have an antiestrogenic activity which selectively inhibits the actions of estrogens on the α-isoform of estrogen receptor. The present invention has been thus completed based on such findings.

In particular, the present invention provides triphenylmethane derivatives (hereinafter referred to as compounds of the present invention) represented by the general formula [I]: 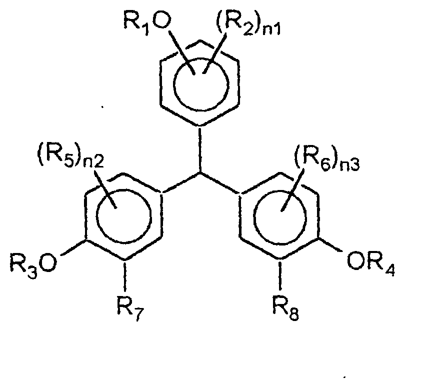 wherein,
R₁ represents a C1-C6-haloalkyl group, a C3-C8-cycloalkyl group, a benzyl group, a C3-C6-alkenyl group, a C3-C6-haloalkenyl group, a C3-C6-alkynyl group, a C3-C6-haloalkynyl group, a cyano-C1-C6-alkyl group, a C1-C4-alkylthio-C1-C4-alkyl group, a (C1-C6-haloalkoxy)carbonyl-C1-C6-alkyl group, a {(C1-C4-alkoxy)C1-C4-alkoxy}carbonyl-C1-C6-alkyl group, a (C3-C8-cycloalkoxy)carbonyl-C1-C6-alkyl group, a (C3-C8-cycloalkyl)C1-C6-alkoxycarbonyl-C1-C6-alkyl group, a C1-C10-alkylsulfinyl-C1-C10-haloalkyl group, a group: -CH₂CON(R₉)R₁₀, -CH(C1-C4-alkyl)CON(R₉)R₁₀, -CH₂CON(R₉)R₁₀, or -CH(C1-C10-alkyl)CON(R₉)R₁₀ (wherein R₉ and R₁₀ are the same or different from each other, and each represent a hydrogen atom, a C1-C6-alkyl group, a C3-C8-cycloalkyl group, a C1-C6-haloalkyl group, a C3-C6-alkenyl group, a C3-C6-alkynyl group, a cyano-C1-C6-alkyl group, a C1-C4-alkoxy-C1-C4-alkyl group, a C1-C4-alkylthio-C1-C4-alkyl group, a carboxy-C1-C6-alkyl group, a (C1-C6-alkoxy)carbonyl-C1-C6-alkyl group, a (C3-C8-cycloalkoxy)carbonyl-C1-C6-alkyl group, a (C1-C6-alkyl)carbonyloxy-C2-C6-alkyl group, a (C1-C6-alkyl)carbonylamino-C2-C6-alkyl group, a hydroxy-C2-C6-alkyl group, an optionally substituted benzyl group, an optionally substituted phenyl group, or a {(C1-C4-alkoxy)C1-C4-alkyl}carbonyl-C1-C6-alkyl group, or R₉ and R₁₀ taken together represent trimethylene, tetramethylene, pentamethylene, ethyleneoxyethylene, ethyleneaminoethylene, or ethylenethioethylene), a group: -CR₁₁R₁₂CR₁₃R₁₄NR₁₅R₁₆ (wherein R₁₁ through R₁₆ are the same or different from each other, and each represent a hydrogen atom or a C1-C6-alkyl group, or R₁₅ and R₁₆ taken together represent trimethylene, tetramethylene, pentamethylene, ethyleneoxyethylene, ethyleneaminoethylene, or ethylenethioethylene), a C2-C6-alkenyloxycarbonyl-C1-C6-alkyl group, a C3-C6-haloalkenyloxycarbonyl-C1-C6-alkyl group, a C3-C6-alkynyloxycarbonyl-C1-C6-alkyl group, a C3-C6-haloalkynyloxycarbonyl-C1-C6-alkyl group, a (C1-C6-alkylthio)carbonyl-C1-C6-alkyl group, a (C1-C6-haloalkylthio)carbonyl-C1-C6-alkyl group, a (C3-C6-alkenylthio)carbonyl-C1-C6-alkyl group, a (C3-C6-haloalkenylthio)carbonyl-C1-C6-alkyl group, a (C3-C6-alkynylthio)carbonyl-C1-C6-alkyl group, a (C3-C6-haloalkynylthio)carbonyl-C1-C6-alkyl group, a (C3-C8-cycloalkylthio)carbonyl-C1-C6-alkyl group, a (C3-C8-cyclohaloalkylthio)carbonyl-C1-C6-alkyl group, a {(C3-C8-cycloalkyl)C1-C6-alkylthio}carbonyl-C1-C6-alkyl group, a di(C1-C6-alkyl)C=NOcarbonyl-C1-C6-alkyl group, an (optionally substituted benzylthio)carbonyl-C1-C6-alkyl group, an (optionally substituted phenylthio)carbonyl-C1-C6-alkyl group, a hydroxy-C2-C6-alkoxycarbonyl-C1-C6-alkyl group, a (C1-C6-alkyl)carbonyloxy-C2-C6-alkoxycarbonyl-C1-C6-alkyl group, a (C1-C6-alkyl)carbonylamino-C2-C6-alkoxycarbonyl-C1-C6-alkyl group, a {(C1-C6-alkoxy)carbonyl-C1-C6-alkyl}oxycarbonyl-C1-C6-alkyl group, a hydroxy-C1-C6-alkyl group, a C1-C6-haloalkoxycarbonyl group, a C3-C8-cycloalkoxycarbonyl group, a C3-C6-alkenyloxycarbonyl group, a benzyloxycarbonyl group, an optionally substituted benzyloxycarbonyl-C1-C6-alkyl group, or an optionally substituted phenoxycarbonyl-C1-C6-alkyl group;
R₂, R₅, and R₆ are the same or different from each other, and each represent a halogen atom, a C1-C6-alkyl group, a C1-C6-haloalkyl group, a C3-C8-cycloalkyl group, a benzyl group, a C3-C6-alkenyl group, a C3-C6-haloalkenyl group, a C3-C6-alkynyl group, a C3-C6-haloalkynyl group, a cyano-C1-C6-alkyl group, a C1-C4-alkoxy-C1-C4-alkyl group, a C1-C4-alkylthio-C1-C4-alkyl group, a carboxy-C1-C6-alkyl group, a (C1-C8-alkoxy)carbonyl-C1-C6-alkyl group, a (C1-C6-haloalkoxy)carbonyl-C1-C6-alkyl group, a hydroxy-C1-C2-alkyl group, a C1-C6-alkoxycarbonyl group, a C1-C6-haloalkoxycarbonyl group, a benzyloxycarbonyl group, or a C1-C6-alkylcarbonyl group, (provided that only R₂ may solely represents a hydroxyl group);
R₃ and R₄ are the same or different from each other, and each represent a hydrogen atom, a C1-C6-alkyl group, a C1-C6-haloalkyl group, a C3-C8-cycloalkyl group, a benzyl group, a C3-C6-alkenyl group, a C3-C6-haloalkenyl group, a C3-C6-alkynyl group, a C3-C6-haloalkynyl group, a cyano-C1-C6-alkyl group, a C1-C4-alkoxy-C1-C4-alkyl group, a C1-C4-alkylthio-C1-C4-alkyl group, a carboxy-C1-C6-alkyl group, a (C1-C8-alkoxy)carbonyl-C1-C6-alkyl group, a (C1-C6-haloalkoxy)carbonyl-C1-C6-alkyl group, a hydroxy-C1-C2-alkyl group, a C1-C6-alkoxycarbonyl group, a C1-C6-haloalkoxycarbonyl group, a benzyloxycarbonyl group, a C1-C6-alkylcarbonyl group, or a R₂₃R₂₄aminocarbonyl group ((R₂₃)R₂₄N-CO), wherein R₂₃ and R₂₄ are the same or different from each other, and each represent a hydrogen atom or a C1-C6-alkyl group, or R₂₃ and R₂₄ taken together represent trimethylene, tetramethylene, pentamethylene, ethyleneoxyethylene, ethyleneaminoethylene, or ethyleneoxyethylene;
R₇ and R₈ are the same or different from each other, and each represent a hydrogen atom, a C1-C6-alkyl group, or a C1-C6-haloalkyl group;
n1 represents 0 to 4;
n2 and n3 are the same or different from each other, and each represent 1 to 3;
provided that when n1 is 2 or above, then respective R₂ may be different from each other; when n2 is 2 or above, then respective R₅ may be different from each other; and when n3 is 2 or above, then respective R₆ may be different from each other;
   and pharmaceutically acceptable salts thereof, as well as pharmaceutical compositions which comprise as an active ingredient a compound represented by the general formula [II]: 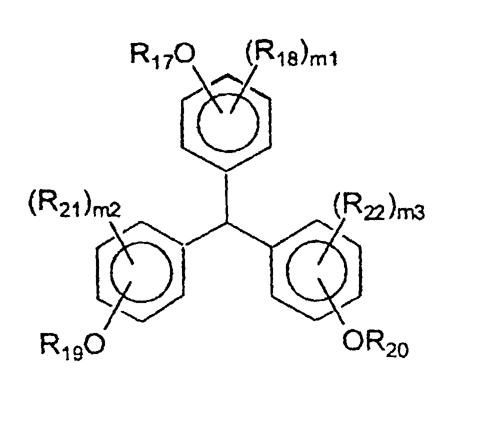 wherein,
[R₁₇, R₁₉, and R₂₀ are the same or different from each other, and each represent a hydrogen atom or an organic residue;
R₁₈, R₂₁, and R₂₂ are the same or different from each other, and each represent a halogen atom, a hydroxyl group, a C1-C6-alkyl group, a C1-C6-haloalkyl group, a C3-C8-cycloalkyl group, a benzyl group, a C3-C6-alkenyl group, a C3-C6-haloalkenyl group, a C3-C6-alkynyl group, a C3-C6-haloalkynyl group, a cyano-C1-C6-alkyl group, a C1-C4-alkoxy-C1-C4-alkyl group, a C1-C4-alkylthio-C1-C4-alkyl group, a carboxy-C1-C-6-alkyl group, a (C1-C8-alkoxy)carbonyl-C1-C6-alkyl group, a (C1-C6-haloalkoxy)carbonyl-C1-C6-alkyl group, a hydroxy-C1-C2-alkyl group, a C1-C6-alkoxycarbonyl group, a C1-C6-haloalkoxycarbonyl group, a benzyloxycarbonyl group, a C1-C6-alkylcarbonyl group, a C1-C6-alkoxy group, a benzyloxy group, a C1-C6-alkylcarbonyloxy group, or a benzoyloxy group;
m1 represents 0 to 4;
m2 and m3 are the same or different from each other, and each represent 1 to 4;
provided that when m1 is 2 or above, then respective R₁₈ may be different from each other; when m2 is 2 or above, then respective R₂₁ may be different from each other; and when m3 is 2 or above, then respective R₂₂ may be different from each other;]
   or a pharmaceutically acceptable salt thereof.

The present invention is described below in more detail.

In connection with compounds of the present invention,
- regarding the group represented by R₁,
   examples of C1-C6-haloalkyl group include 2-chloroethyl, 2-bromoethyl, 2,2,2-trifluoroethyl, and the like;
   examples of C3-C8 cycloalkyl group include cyclopropyl, cyclobutyl, cyclopentyl, cyclohexyl, and the like;
   examples of C3-C6 alkenyl group include allyl, 1-methyl-2-propenyl, 3-butenyl, 2-butenyl, 3-methyl-2-butenyl, 2-methyl-3-butenyl, and the like;
   examples of C3-C6 haloalkenyl group include 2-chloro-2-propenyl, 3,3-dichloro-2-propenyl, and the like;
   examples of C3-C6-alkynyl group include propargyl, 1-methyl-2-propynyl, 2-butynyl, 1,1-dimethyl-2-propynyl, and the like;
   examples of C3-C6-haloalkynyl group include 3-bromopropargyl and the like,
   examples of cyano-C1-C6-alkynyl group include cyanomethyl and the like;
   examples of C1-C4-alkylthio-C1-C4-alkyl group include methylthiomethyl, methylthioethyl, and the like;
   examples of {(C1-C4-alkoxy)C1-C4-alkoxy}carbonyl-C1-C6-alkyl group include methoxymethoxycarbonylmethyl, methoxyethoxycarbonylmethyl, 1-methoxyethoxycarbonylethyl, and the like;
   examples of (C3-C8-cycloalkoxy)carbonyl-C1-C6-alkyl group include cyclobutyloxycarbonylmethyl, cyclopentyloxycarbonylmethyl, cyclohexyloxycarbonylmethyl, 1-cyclobutyloxycarbonylethyl, 1-cyclopentyloxycarbonylethyl, 1 -cyclohexyloxycarbonylethyl, and the like;
   examples of C1-C10-alkylsulfinyl-C1-C10-haloalkyl group include 5,5,5,4,4-pentafluoropentylsulfinylnonyl and the like;
   examples of the group: -CH(C1-C10-alkyl)CON(R₉)R₁₀ include a group: -(CH₂)₁₀CON(CH₃)(CH₂)₃CH₃ and the like;
   examples of C1-C6-haloalkoxycarbonyl group include 2,2,2-trichloroethylcarbonyl and the like;
   examples of C3-C8-cycloalkoxycarbonyl group include cyclopropyloxycarbonyl, cyclobutyloxycarbonyl, and the like;
   examples of C3-C6-alkenyloxycarbonyl group include allyloxycarbonyl and the like; and
   examples of {(C1-C6-alkoxy)carbonyl-C1-C6-alkyl}oxycarbonyl-C1-C6-alkyl group include (methoxycarbonyl)methoxycarbonylmethyl, (ethoxycarbonyl)methoxycarbonylmethyl, and the like:
- examples of C1-C6-alkyl group represented by R₉ or R₁₀ include methyl, ethyl, propyl, isopropyl, and the like:
- examples of C1-C6-alkyl group represented by R₁₁ to R₁₆ include, methyl, ethyl, propyl, isopropyl, isobutyl, butyl, t-butyl wherein "t" indicates "tertiary" : hereinafter it indicates the same meaning wherever it occurs), amyl, isoamyl, t-amyl, and the like:
- the term "halogen atom" represented by R₂, R₅, or R₆ refers to a fluorine, chlorine, bromine, or iodine atom:
- regarding the group represented by R₂, R₃, R₄, R₅, or R₆,
   examples of C1-C6-alkyl group include methyl, ethyl, propyl, isopropyl, isobutyl, butyl, t-butyl, amyl, isoamyl, t-amyl, and the like;
   examples of C1-C6-haloalkyl group include 2-chloroethyl, 2-bromoethyl, 2,2,2-trifluoroethyl, and the like;
   examples of C3-C8-cycloalkyl group include cyclopropyl, cyclobutyl, cyclopentyl, cyclohexyl, and the like;
   examples of C3-C6-alkenyl group include allyl, 1-methyl-2-propenyl, 3-butenyl, 2-butenyl, 3-methyl-2-butenyl, 2-methyl-3-butenyl, and the like;
   examples of C3-C6-haloalkenyl group include 2-chloro-2-propenyl, 3,3-dichloro-2-propenyl, and the like;
   examples of C3-C6-alkynyl group include propargyl, 1-methyl-2-propynyl, 2-butynyl, 1,1-dimethyl-2-propynyl, and the like;
   examples of C3-C6-haloalkynyl group include 3-bromopropargyl and the like;
   examples of cyano-C1-C6-alkyl group include cyanomethyl and the like;
   examples of C1-C4-alkoxy-C1-C4-alkyl group include methoxymethyl, methoxyethyl, ethoxymethyl, ethoxyethyl, and the like;
   examples of C1-C4-alkylthio-C1-C4-alkyl group include methylthiomethyl, methylthioethyl, and the like;
   examples of carboxy-C1-C6-alkyl group include carboxymethyl, 1-carboxyethyl, 2-carboxyethyl, and the like;
   examples of (C1-C8-alkoxy)carbonyl-C1-C6-alkyl group include methoxycarbonylmethyl, ethoxycarbonylmethyl, propoxycarbonylmethyl, isopropoxycarbonylmethyl, butoxycarbonylmethyl, isobutoxycarbonylmethyl, t-butoxycarbonylmethyl, amyloxycarbonylmethyl, isoamyloxycarbonylmethyl, t-amyloxycarbonylmethyl, 1-methoxycarbonylethyl, 1-ethoxycarbonylethyl, 1-propoxycarbonylethyl, 1-isopropoxycarbonylethyl, 1-butoxycarbonylethyl, 1-isobutoxycarbonylethyl, 1-t-butoxycarbonylethyl, 1-amyloxycarbonylethyl, 1-isopropoxycarbonylethyl, 1-t-butoxycarbonylethyl, 1-t-amyloxycarbonylethyl, 1-isoamyloxycarbonylethyl, 1-t-amyloxycarbonylethyl, and the like;
   examples of (C1-C6-haloalkoxy)carbonyl group include 2-chloroethoxycarbonyl, 2-bromoethoxycarbonyl, 3-chlorobutoxycarbonyl, 1-chloro-2-propoxycarbonyl, 1,3-dichloro-2-propoxycarbonyl, 2,2-dichloroethoxycarbonyl, 2,2,2-trifluoroethoxycarbonyl, 2,2,2-trichloroethoxycarbonyl, 2,2,2-tribromoethoxycarbonyl, and the like;
   examples of hydroxy-C1-C2-alkyl group include hydroxymethyl, hydroxyethyl, and the like;
   examples of C1-C6-alkoxycarbonyl group include methoxycarbonyl, ethoxycarbonyl, propoxycarbonyl, butoxycarbonyl, isopropoxycarbonyl, isobutoxycarbonyl, t-butoxycarbonyl, and the like; and
   examples of C1-C6-haloalkoxycarbonyl group include 2,2,2-trichloroethylcarbonyl and the like:
- examples of R₂₃R₂₄ aminocarbonyl group represented by R₃ or R₄ include dimethylaminocarbonyl and the like:
- regarding the groups represented by R₇ and R₈,
   examples of C1-C6-alkyl group include methyl, ethyl, propyl, isopropyl, isobutyl, butyl, t-butyl, amyl, isoamyl, t-amyl, and the like; and
   examples of C1-C6-haloalkyl group include 2-chloroethyl, 2-bromoethyl, 2,2,2-trifluoroethyl, and the like.

In connection with compounds represented by the general formula [II], the organic residues represented by R₁₇, R₁₉, and R₂₀ may be any of those organic residues capable of binding to oxygen. Examples of such organic residue may include the following groups:
C1-C6-alkyl, C1-C6-haloalkyl, C3-C8-cycloalkyl, benzyl, C3-C6-alkenyl, C3-C6-haloalkenyl, C3-C6-alkynyl, C3-C6-haloalkynyl, cyano-C1-C6-alkyl, C1-C4-alkoxy-C1-C4-alkyl, C1-C4-alkylthio-C1-C4-alkyl, carboxy-C1-C6-alkyl, (C1-C8-alkoxy)carbonyl-C1-C6-alkyl, (C1-C6-haloalkoxy)carbonyl-C1-C6-alkyl, {(C1-C4-alkoxy)C1-C4-alkoxy}carbonyl-C1-C6-alkyl, (C3-C8-cycloalkoxy)carbonyl-C1-C6-alkyl, (C3-C8-cycloalkyl)C1-C6-alkoxycarbonyl-C1-C6-alkyl, C1-C10-alkylsulfinyl-C1-C10-haloalkyl, -CH₂CON(R₉)R₁₀, -CH(C1-C4-alkyl)CON(R₉)R₁₀, -CH₂CON(R₉)R₁₀, -CH(C1-C10-alkyl)CON(R₉)R₁₀, (wherein R₉ and R₁₀ are as defined above,) -CR₁₁R₁₂CR₁₃R₁₄NR₁₅R₁₆, (wherein R₁₁ through R₁₆ are as defined above,) C2-C6-alkenyloxycarbonyl-C1-C6-alkyl, C3-C6-haloalkenyloxycarbonyl-C1-C6-alkyl, C3-C6-alkynyloxycarbonyl-C1-C6-alkyl, C3-C6-haloalkynyloxycarbonyl-C1-C6-alkyl, (C1-C6-alkylthio)carbonyl-C1-C6-alkyl, (C1-C6-haloalkylthio)carbonyl-C1-C6-alkyl, (C3-C6-alkenylthio)carbonyl-C1-C6-alkyl, (C3-C6-haloalkenylthio)carbonyl-C1-C6-alkyl, (C3-C6-alkynylthio)carbonyl-C1-C6-alkyl, (C3-C6-haloalkynylthio)carbonyl-C1-C6-alkyl, (C3-C8-cycloalkylthio)carbonyl-C1-C6-alkyl, (C3-C8-cyclohaloalkylthio)carbonyl-C1-C6-alkyl, {(C3-C8-cycloalkyl)C1-C6-alkylthio}carbonyl-C1-C6-alkyl, di(C1-C6-alkyl)C=NOcarbonyl-C1-C6-alkyl, (optionally substituted benzylthio)carbonyl-C1-C6-alkyl, (optionally substituted phenylthio)carbonyl-C1-C6-alkyl, hydroxy-C2-C6-alkoxycarbonyl-C1-C6-alkyl, (C1-C6-alkyl)carbonyloxy-C2-C6-alkoxycarbonyl-C1-C6-alkyl, (C1-C6-alkyl)carbonylamino-C2-C6-alkoxycarbonyl-C1-C6-alkyl, {(C1-C6-alkoxy)carbonyl-C1-C6-alkyl}oxycarbonyl-C1-C6-alkyl, hydroxy-C1-C6-alkyl, C1-C6-haloalkoxycarbonyl, C3-C8-cycloalkoxycarbonyl, C3-C6-alkenyloxycarbonyl, benzyloxycarbonyl, optionally substituted benzyloxycarbonyl-C1-C6-alkyl, optionally substituted phenoxycarbonyl-C1-C6-alkyl, R₂₃R₂₄ aminocarbonyl (wherein R₂₃ and R₂₄ are as defined above,) and the like.

In connection with R₁₈, R₂₁, and R₂₃:
the term "halogen atom" refers to a fluorine, chlorine, bromine, or iodine atom;
examples of C1-C6-alkyl group include methyl, ethyl, propyl, isopropyl, isobutyl, butyl, t-butyl, amyl, isoamyl, t-amyl, and the like;
examples of C1-C6-haloalkyl group include 2-chloroethyl, 2-bromoethyl, 2,2,2-trifluoroethyl, and the like;
examples of C3-C8 cycloalkyl group include cyclopropyl, cyclobutyl, cyclopentyl, cyclohexyl, and the like;
examples of C3-C6 alkenyl group include allyl, 1-methyl-2-propenyl, 3-butenyl, 2-butenyl, 3-methyl-2-butenyl, 2-methyl-3-butenyl, and the like;
examples of C3-C6 haloalkenyl group include 2-chloro-2-propenyl, 3,3-dichloro-2-propenyl, and the like;
examples of C3-C6-alkenyl group include propargyl, 1-methyl-2-propynyl, 2-butynyl, 1,1-dimethyl-2-propynyl, and the like;
examples of C3-C6-haloalkynyl group include 3-bromopropargyl and the like;
examples of cyano-C1-C6-alkyl group include cyanomethyl and the like;
examples of C1-C4-alkoxy-C1-C4-alkyl group include methoxymethyl, methoxyethyl, ethoxymethyl, ethoxyethyl, and the like;
examples of C1-C4-alkylthio-C1-C4-alkyl group include methylthiomethyl, methylthioethyl, and the like;
examples of carboxy-C1-C6-alkyl group include carboxymethyl, 1-carboxyethyl, 2-carboxyethyl, and the like;
examples of (C1-C8-alkoxy)carbonyl-C1-C6-alkyl group include methoxycarbonylmethyl, ethoxycarbonylmethyl, propoxycarbonylmethyl, isopropoxycarbonylmethyl, butoxycarbonylmethyl, isobutoxycarbonylmethyl, t-butoxycarbonylmethyl, amyloxycarbonylmethyl, isoamyloxycarbonylmethyl, t-amyloxycarbonylmethyl, 1-methoxycarbonylethyl, 1-ethoxycarbonylethyl, 1-propoxycarbonylethyl, 1-isopropoxycarbonylethyl, 1-butoxycarbonylethyl, 1-isobutoxycarbonylethyl, 1-t-butoxycarbonylethyl, 1-amyloxycarbonylethyl, 1-isoamyloxycarbonylethyl, 1-t-butoxycarbonylethyl, 1-amyloxycarbonylethyl, 1-isoamyloxycarbonylethyl, 1-t-amyloxycarbonylethyl, and the like;
examples of (C1-C6-haloalkoxy)carbonyl group include 2-chloroethoxycarbonyl, 2-bromoethoxycarbonyl, 3-chlorobutoxycarbonyl, 1-chloro-2-propoxycarbonyl, 1,3-dichloro-2-propoxycarbonyl, 2,2-dichloroethoxycarbonyl, 2,2,2-trifluoroethoxycarbonyl, 2,2,2-trichloroethoxycarbonyl, 2,2,2-tribromoethoxycarbonyl, and the like;
examples of hydroxy-C1-C2-alkyl include hydroxymethyl, hydroxyethyl, and the like;
examples of C1-C6-alkoxycarbonyl group include methoxycarbonyl, ethoxycarbonyl, propoxycarbonyl, butoxycarbonyl, isopropoxycarbonyl, isobutoxycarbonyl, t-butoxycarbonyl, and the like;
examples of C1-C6-haloalkoxycarbonyl group include 2,2,2-trichloroethylcarbonyl and the like.

Compounds of the present invention or compounds represented by the general formula [II] may exist as geometric isomers due to double bond(s), optical isomers due to asymmetric carbon atom(s), and/or diastereomers, and such respective isomers and mixtures thereof are included within the present invention.

Examples of pharmaceutically acceptable salt of a compound of the present invention or of a compound represented by the general formula [II] may include the acid addition salts such as those with hydrochloric acid, hydrobromic acid, hydriodic acid, nitric acid, phosphoric acid, formic acid, acetic acid, beuzoic acid, maleic acid, fumaric acid, succinic acid, tartaric acid, citric acid, oxalic acid, glyoxylic acid, aspartic acid, methanesulfonic acid, ethanesulfonic acid, or beuzenesulfonic acid, as well as alkali metal salts such as lithium salt, sodium salt, and potassium salt of the phenolic hydroxyl group(s).

In the following paragraphs, methods for preparing compounds of the present invention or compounds represented by the general formula [II] are described.

A compound of the present invention or compound represented by the general formula [II] may be obtained by reacting a benzaldehyde derivative represented by the general formula [III]: 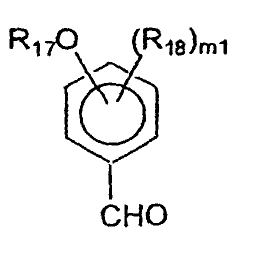 [wherein R₁₇, R₁₈, and m1 are as defined above,]
with benzene derivatives represented by the general formula [IV]: 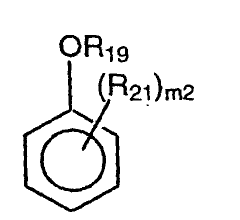 and the general formula [V]: 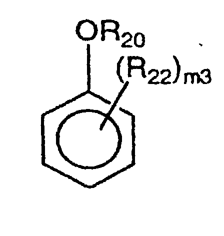 [wherein R₁₉ through R₂₂, m2, and m3 are as defined above,]
in a solvent in the presence of an acid.

Such benzene derivatives are used in a two fold mole amount or more, usually in a 2- to 10-fold mole amount, and preferably in a 3- to 6-fold mole amount, relative to the above benzaldehyde derivative. These benzene derivatives are used alone, or as a mixture of two or more kinds of such derivatives.

A benzaldehyde derivative of formula [III] may be commercially available, or may be prepared according to the procedures, for example, described in Houben-Weyl, *Methods of Organic Chemistry,* Vol VII (1975) and vol. E3 (1983). Compounds of formula [IV] and [V] may be commercially available, or may be prepared in the usual manner from phenolic compounds which are obtainable by the methods described in Vol. V/1c (1976) of the series of references mentioned above.

In the above method for preparing compounds of the present invention or compounds represented by the general formula [II], reaction solvents used are preferably lower aliphatic alcohols having 1 to 4 carbon atoms such as methanol, ethanol, isopropyl alcohol, n-propyl alcohol, t-butyl alcohol, isobutyl alcohol, n-butyl alcohol. Such alcohol solvents are typically used in an amount of 10 to 1000 parts by weight, and preferably 100 to 500 parts by weight, per 100 parts by weight of the benzaldehyde derivative.

Acids used in the above preparation process are preferably those acids which are soluble in the alcohol used as the reaction solvent, such as hydrochloric acid, sulfuric acid, sulfuric anhydride, formic acid, phosphoric acid, oxalic acid, p-toluenesulfonic acid, methanesulfonic acid, trifluoromethanesulfonic acid, trichloro acetate and trifluoro acetate. Such acids are used in an amount of 1 to 10 moles per mole of the benzaldehyde derivative.

In order to achieve the purpose of the present invention, the reaction may be typically conducted at a temperature between room temperature and 80°C, preferably in the range of 30 to 60°C, with stirring for about 2 to 24 hours, typically for about 10 to 15 hours.

After completion of the reaction, the product of interest may be isolated by collecting the crystals formed after, if necessary, adding water to the reaction mixture, or by working up in the usual manner, for example, using extraction with organic solvent(s) and/or concentration, followed by, if necessary, procedures such as chromatography and/or recrystallization.

The substituents R₁₉ and R₂₀ in compounds of the present invention or compounds represented by the general formula [II] may also be introduced after conducting the reaction using compounds represented by the general formulas 6 and 7 which contain free hydroxyl groups (*i.e*., those compounds wherein R₁₉=R₂₀=H), by reacting the product with an appropriate halogenated compound. For example, carbamoyl groups may be introduced by reacting the product with dimethylcarbamoyl chloride under basic conditions (e.g. in the presence of triethylamine or NaH).

Of pharmaceutically acceptable salts of compounds of the present invention and of compounds represented by the general formula [II], an acid addition salt may be obtained by reacting a compound of the present invention or a compound represented by the general formula [II] with an acid such as hydrochloric acid, hydrobromic acid, hydriodic acid, nitric acid, phosphoric acid, formic acid, acetic acid, benzoic acid, maleic acid, fumaric acid, succinic acid, tartaric acid, citric acid, oxalic acid, glyoxylic acid, aspartic acid, methanesulfonic acid, ethanesulfonic acid, or benzenesulfonic acid. Similarly, an alkali metal salt may be obtained by reacting a compound of the present invention or a compound represented by the general formula [II] with an alkali metal hydroxide such lithium hydroxide, sodium hydroxide, or potassium hydroxide. Pharmaceutically acceptable salts of compounds of the present invention or of compounds represented by the general formula [II] prepared in this manner may be purified, if necessary, by recrystallization or other procedures.

Typical examples of compound represented by the general formula [II] are shown below.

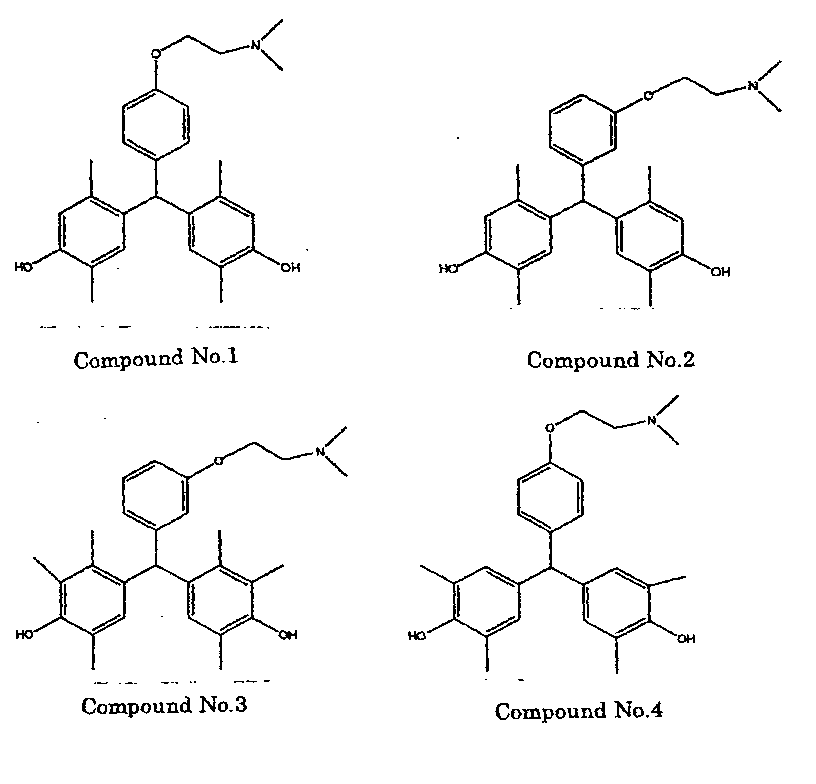

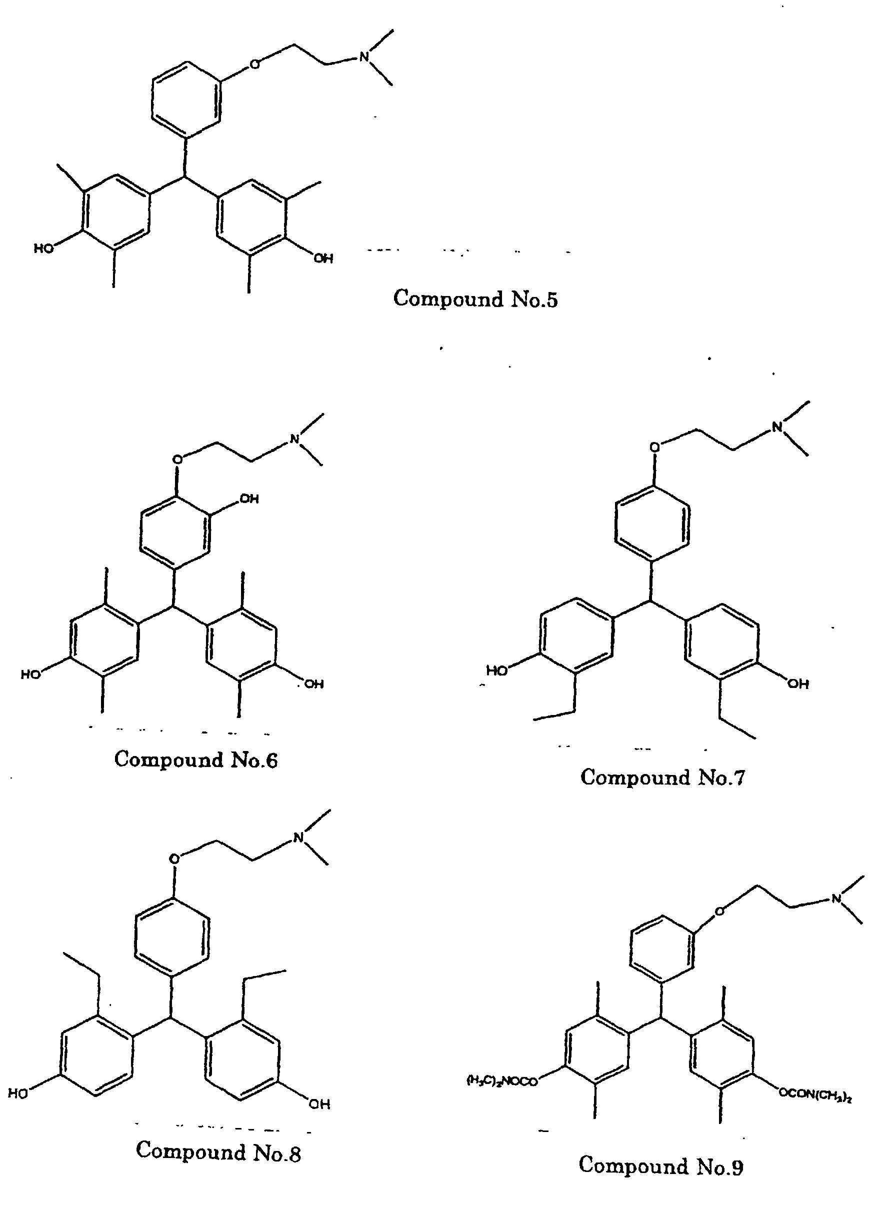

Compounds of the present invention and compounds represented by the general formula [II] have an antiestrogenic activity of selectively inhibiting the action of estrogens on the α-isoform of estrogen receptor. They are therefore useful as remedies for estrogen dependent diseases such as breast cancer and endometrial cancer. In addition, those compounds of the present invention or compounds represented by the general formula [II] which have an activity of promoting differentiation of osteoblast are useful for treatment or prevention of osteoporosis as drugs having an osteogenic activity, and those compounds of the present invention and compounds represented by the general formula [II] which have an activity of inhibiting the decrease in bone mineral density caused by deficiency of estrogens are useful for treatment of osteoporosis.

When used as pharmaceutical agents or animal drugs, compounds of the present invention and compounds represented by the general formula [II] are used in the form of appropriate formulations such as tablets, capsules, injections, drops, and suppository prepared by the usual preparation methods. Such dosage forms are formulated using generally known procedures, and may further contain, for example, various excipients, lubricants, binders, disintegrators, and/or isotonizing agents. Dosing methods for such formulations are not specifically restricted, and may be by oral administration, intravenous injection, intramuscular injection, drip phleboclysis, intrarectal administration, or through other routes. Although the dose may vary depending on the type of compound, the dosing method, symptoms and age of the patients, and other factors, it is typically administered in an amount in the range of 0.001 to 500 mg/kg-weight/day at a frequency of once to several times a day or once every other day to once every few days. Such dose may be, however, increased or decreased as appropriate if necessary.

### Examples

The present invention is described in more detail by making reference to the following Examples and Experiments. The present invention is, however, not restricted thereto.

### Example 1: Preparation of bis(2,5-dimethyl-4-hydroxyphenyl)[4-(2-dimethylamino-1-ethoxy)phenyl]methane (Compound 1)

Dimethylformamide (2 ml), 4-hydroxybenzaldehyde (0.5 g, 4.094 mmol), potassium carbonate (1.23 g, 8.90 mmol), 1-dimethylamino-2-chloroethane (0.66g, 6.18 mmol), and diisopropyl ether (0.25 ml) were introduced into a reaction vessel, and stirred at 60°C for 1 hour and 30 minutes. After standing to cool, water was added to the reaction mixture, and the mixture was extracted with ethyl acetate. The organic phase was washed with saturated saline solution, dried over magnesium sulfate, and the solvent was evaporated. The resultant residue (0.77 g) was distilled under reduced pressure (110°C/1-3 mmHg) to yield 4-(2-dimethylamino-1-ethoxy)benzaldehyde (0.45 g, 57%).

4-(2-Dimethylamino-1-ethoxy)benzaldehyde (0.15 g, 0.776 mmol) was mixed with 2,5-dimethylphenol (0.227 g, 1.86 mmol), methanol (0.4 g) and 35% aqueous hydrochloric acid (40 mg), stirred at 40°C for 10 hours. To the reaction mixture, 35% aqueous hydrochloric acid (85 mg) was further added, and stirred at 60°C for 8 hours and 30 minutes. The reaction was then left overnight to cool, during which crystals precipitated. To the semicrystals collected by decantation, 1.5 ml of hexane was added, and decanted. Furthermore, 1.5 ml of ethyl acetate was added, and stirred. Crystals precipitated were then filtered, and washed (twice with each 1 ml of ethyl acetate, and once with 1 ml of hexane) to yield crystals (0.333 g, 94%) of the aimed product, bis(2,5-dimethyl-4-hydroxyphenyl)[4-(2-dimethylamino-1-ethoxy)phenyl]methane (Compound 1): ¹NMR [270 MHz, CDCl₃, TMS, δ (ppm)] 2.03 (6H, s), 2.04 (6H, s), 2.93 (6H, s), 3.58 (2H, m), 4.32 (2H, m), 5.43 (1H, s), 6.37 (2H, s), 6.59 (2H, s), 6.87-6.98 (4H, m).

### Example 2: Preparation of bis(2,5-dimethyl-4-hydroxyphenyl)[3-(2-dimethylamino-1-ethoxy)phenyl]methane (Compound 2)

Dimethylformamide (24 ml), 3-hydroxybenzaldehyde (6.0 g, 49.1 mmol), potassium carbonate (14.8 g, 107 mmol), 1-dimethylamino-2-chloroethane (7.92 g, 73.7 mmol), and diisopropyl ether (3 ml) were introduced into a reaction vessel, stirred at 60°C for 1 hour and 30 minutes, at 80°C for 3 hours and 30 minutes, and further at 70°C for 2 hours. After standing to cool, water was added to the reaction mixture, and the mixture was extracted with ethyl acetate. The organic phase was washed with saturated saline solution, and dried over magnesium sulfate. The residue (8.6 g) obtained by evaporating the solvent was purified by silica gel column chromatography (86 g silica gel, elution solvent: ethyl acetate/methanol=5/2 (v/v)) to obtain the aimed product 3-(2-dimethylamino-1-ethoxy)benzaldehyde (6.35 g, 67%).

3-(2-Dimethylamino-1-ethoxy)benzaldehyde (1.5 g, 7.76 mmol) was mixed with 2,5-dimethylphenol (2.37 g, 19.4 mmol), methanol (4 g) and 35% aqueous hydrochloric acid (1.2 g), and stirred at 60°C for 3 hours. After standing at room temperature for 10 hours, the precipitated crystals were collected by filtration, and washed (twice with each 2 ml of cold methanol, and twice with each 2 ml of ether). To the crude crystals thus obtained, 4.5 ml of methanol was added, stirred at 60°C, and allowed to cool. The crystals thus obtained were filtered again, washed (twice with each 1.5 ml of cold methanol, twice with each 2 ml of ether, and twice with each 2 ml of hexane), and dried to obtain crystals (1.43 g, 40.5%) of bis(2,5-dimethyl-4-hydroxyphenyl)[3-(2-dimethylamino-1-ethoxy)phenyl]methane (Compound 2): ¹H NMR [270 MHz, CDCl₃, TMS, δ (ppm)] 2.01 (6H, s), 2.02 (6H, s), 2.93 (6H, s), 3.53 (2H, t, J=5.0 Hz), 4.26 (2H, t, J=5.0 Hz), 5.47 (1H, s), 6.35 (2H, s), 6.58-6.62 (3H, m), 6.69 (1H, m), 6.85 (1H,m), 7.22 (1H, m).

### Experiment 1: Antiestrogenic activity

cDNA fragments for α- and β-isoforms of human estrogen receptor were cloned from a human liver cDNA library (Toyobo Co, Ltd.) by RT-PCR using oligonucleotides having the following base sequences as primers. For cloning the α-isoform, oligonucleotides 5'-CGGGAAGCCAATCTGTACCT-3' and 5'-CAGGGATTCGCAGAACCTTG-3' were used, while oligonucleotides 5'-ATGAATTACAGCATTCCCAG-3' and 5'-AAATGAGGGACCACACAGCA-3' were used for cloning the β-isoform. The cDNA fragments for the α- and β-isoforms obtained by the RT-PCR procedures were blunt-ended, and each inserted into a vector for expression pRc/RSV (Invitrogen) at its blunt-ended *Hin*d III cleavage site to obtain expression plasmids pRc-RSV-hERα and pRc-RSV-hERβ. In addition, an oligonucleotide 5'-AAAGTCAGGTCACAGTGACCTGATCA-3' comprising an estrogen response element was inserted into a vector pGV-P (Toyo Ink Mfg. Co., Ltd.) at the *Sma* I cleavage site to obtain a luciferase reporter plasmid pGV-ERE.

Separately, HeLa cells which had been subcultured in Eagle's MEM medium containing 10% (v/v) phenol red-free and active charcoal-treated serum were inoculated in 24-well plates (⌀ 16 mm well) at a density of 40,000 cells/well, cultured for 24 hours at 37°C in the presence of 5% CO₂, and then the medium was exchanged with serum-free Eagle's MEM medium.

The above expression plasmid pRc-RSV-hERα or pRc-RSV-hERβ, and the luciferase reporter plasmid pGV-ERE incorporating the estrogen response element were transfected into the above HeLa cells using the lipofectin method. Specifically, 0.3 µg of the expression plasmid pRc-RSV-hERα or pRc-RSV-hERβ, 0.1 µg of pGV-ERE, and 0.35 µl of Lipofectin mixed together, and added to the above HeLa cells. The HeLa cells were then cultured for 6 hours at 37°C in the presence of 5% CO₂. Next, the medium was exchanged with Eagle's MEM medium containing 10% serum (active charcoal-treated), and the cultivation was continued overnight at 37°C under 5% CO₂. Furthermore, the medium was exchanged with Eagle's MEM medium containing 10% serum (active charcoal-treated) which had been further supplemented with a solution of a test compound in DMSO (or DMSO alone) and 17β-estradiol (final concentration: 100 pM for the α-isoform, and 1 nM for the β-isoform), and the cells were cultured for 48 hours. After removing the medium and washing the cells twice with PBS(-), 50 µl/well of a lysis solution was added, and the cells were lysed at room temperature. An aliquot of this cell lysate was mixed with a luciferase assay substrate solution (Toyo Ink Mfg. Co., Ltd.), and the luminescence was measured using a luminometer. For each of α- and β-isoforms of estrogen receptor, the relative value of luminescence obtained in each experiment in which 17β-estradiol and a solution of one of test compounds in DMSO were added was then calculated, considering the luminescence obtained when 17β-estradiol and DMSO were added as 100. Next, the antiestrogenic activity (β/α) was calculated by dividing the relative value thus obtained for the β-isoform by that for the α-isoform.

**Table 1**

| Compound | Antiestrogenic activity (β/α) | | |
|---|---|---|---|
| | Concentration of compound | | |
| | 0.1 µM | 1 µM | 10 µM |
| Compound 1 | 5.2 | 8.6 | 3.9 |
| Compound 2 | 19 | 5.5 | 2.3 |
| Compound 3 | 2.6 | 4.0 | 2.0 |
| Compound 4 | 1.5 | 14 | 5.0 |
| Compound 5 | - | 8.4 | 5.0 |
| Compound 6 | 2.9 | 4.5 | 10.1 |
| Compound 7 | 2.9 | 7.9 | 4.2 |
| Compound 8 | 0.9 | 1.7 | 5.6 |
| Hydroxytamoxifen | 1.3 | 1.9 | 1.5 |

### Experiment 2: Effects on osteoblast

Rat osteoblastic cell line UMR 106 was subcultured in Eagle's MEM medium containing 10% fetal bovine serum (active charcoal-treated), inoculated in 6-well plates (⌀ 36 mm well) at a density of 20,000 cells/well, and cultured overnight in 2 ml/well of the same serum-containing Eagle's MEM medium at 37°C in the presence of 5% CO₂. On the following day, the medium was aspirated, and the wells were washed by adding 1 ml/well of serum-free Eagle's MEM medium supplemented with Supplement A (a solution of insulin, transferrin, and selenium; Gibco BRL). After aspirating the washings, 2 ml/well of serum-free Eagle's MEM medium supplemented with Supplement A was newly added, and the cultivation was further continued at 37°C. Two days after the medium exchange, the medium was aspirated from each well. Into the wells, 2 ml/well of serum-free Eagle's MEM medium containing Supplement A to which a solution of a test compound in DMSO (or DMSO alone) had been added and mixed was then added, and the cultivation was further continued at 37°C. Two days after the addition of compound, the medium was collected, centrifuged (14,000 rpm, 5 minutes), and the supernatant was aliquoted. To each 0.6 ml aliquot, 20 µl of 1N HCl was added, mixed and then they were stored with ice-cooling. On the other hand, the wells from which the medium had been removed were washed twice with PBS (-). After thoroughly removing the residual liquid, 0.5 ml/well of extra pure water was added, and the cells were harvested, and stored at -80°C. The ice-cold medium supernatant described above was neutralized with 1N NaOH, and 100 µl aliquot thereof was sampled, and used in quantifying TGFβ using an ELISA kit for TGFβ (Biochemical Research institute, Morinaga Milk Industry Co. Ltd.). On the other hand, the frozen cells were thawed by bringing them to room temperature. The cell suspension thus obtained by thawing the frozen cells was then centrifuged (14,000 rpm, 5 minutes), and 50 µl of the supernatant was used in measurement of alkaline phosphatase (ALP) activity. ALP activity was measured according to the method of Lowry [*J. Biol. Chem.* vol. 207, 19-37 (1954)]. The samples remained without being used for measurements of TGFβ and ALP activity were used in quantification of protein according to the method of Lowry [*J. Biol. Chem.* vol. 193, 265-275 (1951)].

The relative values for the amount of TGFβ and the ALP activity after correcting for the amounts of protein are shown in Table 2.

**Table 2**

| | ALP activity* | TGFβ* |
|---|---|---|
| Control (DMSO) | 1 | 1 |
| Compound 1 0.1 µM | 2.0 | 1.4 |
| Compound 1 1 µM | 5.4 | 1.1 |
| Compound 2 0.1 µM | 1.5 | - |

| | | |
|---|---|---|
| * Relative values considering the value for control as 1 (absorbance/mg-protein/ml) | | |

### Experiment 3: Effects on ovariectomized rat

### (1) Ovariectomy

Six-weeks old female SD rats (Charles River Japan) were subjected to ovariectomy of right and left ovaries, after one-week prefeeding. Firstly, the hairs around the both flanks of the rats were shaved away. After anesthetizing the rat with diethyl ether, the area around the flanks was disinfected, and incised about 1 cm below the last ribs. The ovaries and uterus were extracted along with fats through the incision, and the ovaries were excised after ligating between ovaries and uterus with suture. The uterus and fats were then returned into the abdomens, and the incision was sutured. For rats receiving mock operations, ovaries were extracted as described above, and then returned into the abdomens as they were without ligature and excision. The rats which received such operations were divided into groups of four animals, and each group was housed in a polypropylene cage, and fed with chow (CRF-1; Charles River Japan) and water ad libitum.

### (2) Preparation of liquids for administration, administration, and measurement of bone mineral density

A compound was weighed out into a mortar, and the crystals were finely ground with a pestle. To the ground compound, 1% hydroxypropyl cellulose (HPC, 150-400 cP) was then added in small amounts while rubbing for suspending and mixing the compound to prepare an administration liquid having a given concentration of the compound. Such liquids for administration were newly prepared every week.

The weights of ovariectomized rats described above were measured at an interval of one week, and the doses of the above liquid for administration were determined based on the weights. The same doses were used for one week until the next weight measurement. Administration to rats was started at one week after the ovariectomy, and the above liquids for administration were orally administered 17 times in total to groups of 5-8 animals through a 2 ml volume syringe attached with a metal sonde and once a day at a frequency of 5 times a week. On the day following the last administration, each rat was exsanguinated from the abdominal aorta, and the tibias were removed, under anesthesia with diethyl ether. The bone mineral density of the tibial epiphysis proximal to the knee was measured by an area scanning method. The results are shown in Table 3.

**Table 3**

| Rat | Compound administered | Dose of compound (mg-compound/kg-weight/day)^{a} | Bone mineral density (mg/cm²)^{b} |
|---|---|---|---|
| mock operated | 1% HPC | 0 | 103.1* |
| ovariectomized | 1% HPC | 0 | 79.9 |
| ovariectomized | ethinyl estradiol | 0.1 | 88.6* |
| ovariectomized | Compound 1 | 50 | 88.0* |
| ovariectomized | Compound 2 | 50 | 91.2* |
| ovariectomized | Compound 6 | 50 | 88.0* |

| | | | |
|---|---|---|---|
| ^{a} administered volume: 5 ml/kg-weight/day | | | |
| ^{b} average for 5-8 animals | | | |
| * statistically different when compared to the control group (ovariectomized rats administered 1% HPC) (*P*<0.05) | | | |

### Industrial Applicability

The novel triphenylmethane derivatives and pharmaceutically acceptable salts thereof provided by the present invention have an antiestrogenic activity of selectively inhibiting the action of estrogens on the α-isoform of estrogen receptor, and thus are useful as remedies for estrogen dependent diseases. Furthermore, since the pharmaceutical compositions provided by the present invention comprise, as an active ingredient, a triphenylmethane derivative or pharmaceutically acceptable salt thereof having an antiestrogenic activity of selectively inhibiting the action of estrogens on the α-isoform of estrogen receptor, they serve as remedies for estrogen dependent diseases.

## Claims

1. A triphenylmethane derivative represented by the general formula [I]: 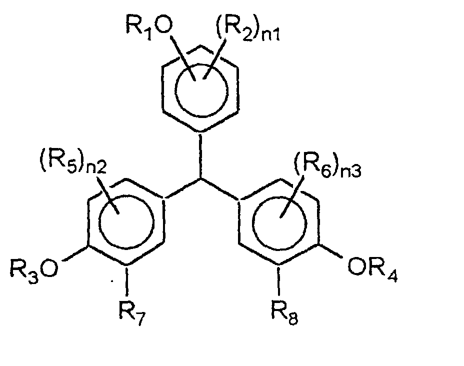 wherein,
R₁ represents a C1-C6-haloalkyl group, a C3-C8-cycloalkyl group, a benzyl group, a C3-C6-alkenyl group, a C3-C6-haloalkenyl group, a C3-C6-alkynyl group, a C3-C6-haloalkynyl group, a cyano-C1-C6-alkyl group, a C1-C4-alkylthio-C1-C4-alkyl group, a (C1-C6-haloalkoxy)carbonyl-C1-C6-alkyl group, a {(C1-C4-alkoxy)C1-C4-alkoxy}carbonyl-C1-C6-alkyl group, a (C3-C8-cycloalkoxy)carbonyl-C1-C6-alkyl group, a (C3-C8-cycloalkyl)C1-C6-alkoxycarbonyl-C1-C6-alkyl group, a C1-C10-alkylsulfinyl-C1-C10-haloalkyl group, a group: -CH₂CON(R₉)R₁₀, -CH(C1-C4-alkyl)CON(R₉)R₁₀, -CH₂CON(R₉)R₁₀, or -CH(C1-C10-alkyl)CON(R₉)R₁₀ (wherein R₉ and R₁₀ are the same or different from each other, and each represent a hydrogen atom, a C1-C6-alkyl group, a C3-C8-cycloalkyl group, a C1-C6-haloalkyl group, a C3-C6-alkenyl group, a C3-C6-alkynyl group, a cyano-C1-C6-alkyl group, a C1-C4-alkoxy-C1-C4-alkyl group, a C1-C4-alkylthio-C1-C4-alkyl group, a carboxy-C1-C6-alkyl group, a (C1-C6-alkoxy)carbonyl-C1-C6-alkyl group, a (C3-C8-cycloalkoxy)carbonyl-C1-C6-alkyl group, a (C1-C6-alkyl)carbonyloxy-C2-C6-alkyl group, a (C1-C6-alkyl)carbonylamino-C2-C6-alkyl group, a hydroxy-C2-C6-alkyl group, an optionally substituted benzyl group, an optionally substituted phenyl group, or a {(C1-C4-alkoxy)C1-C4-alkyl}carbonyl-C1-C6-alkyl group, or R₉ and R₁₀ taken together represent trimethylene, tetramethylene, pentamethylene, ethyleneoxyethylene, ethyleneaminoethylene, or ethylenethioethylene), a group: -CR₁₁R₁₂CR₁₃R₁₄NR₁₅R₁₆ (wherein R₁₁ through R₁₆ are the same or different from each other, and each represent a hydrogen atom or a C1-C6-alkyl group, or R₁₅ and R₁₆ taken together represent trimethylene, tetramethylene, pentamethylene, ethyleneoxyethylene, ethyleneaminoethylene, or ethylenethioethylene), a C2-C6-alkenyloxycarbonyl-C1-C6-alkyl group, a C3-C6-haloalkenyloxycarbonyl-C1-C6-alkyl group, a C3-C6-alkynyloxycarbonyl-C1-C6-alkyl group, a C3-C6-haloalkynyloxycarbonyl-C1-C6-alkyl group, a (C1-C6-alkylthio)carbonyl-C1-C6-alkyl group, a (C1-C6-haloalkylthio)carbonyl-C1-C6-alkyl group, a (C3-C6-alkenylthio)carbonyl-C1-C6-alkyl group, a (C3-C6-haloalkenylthio)carbonyl-C1-C6-alkyl group, a (C3-C6-alkynylthio)carbonyl-C1-C6-alkyl group, a (C3-C6-haloalkynylthio)carbonyl-C1-C6-alkyl group, a (C3-C8-cycloalkylthio)carbonyl-C1-C6-alkyl group, a (C3-C8-cyclohaloalkylthio)carbonyl-C1-C6-alkyl group, a {(C3-C8-cycloalkyl)C1-C6-alkylthio)carbonyl-C1-C6-alkyl group, a di(C1-C6-alkyl)C=NOcarbonyl-C1-C6-alkyl group, an (optionally substituted benzylthio)carbonyl-C1-C6-alkyl group, an (optionally substituted phenylthio)carbonyl-C1-C6-alkyl group, a hydroxy-C2-C6-alkoxycarbonyl-C1-C6-alkyl group, a (C1-C6-alkyl)carbonyloxy-C2-C6-alkoxycarbonyl-C1-C6-alkyl group, a (C1-C6-alkyl)carbonylamino-C2-C6-alkoxycarbonyl-C1-C6-alkyl group, a {(C1-C6-alkoxy)carbonyl-C1-C6-alkyl}oxycarbonyl-C1-C6-alkyl group, a hydroxy-C1-C6-alkyl group, a C1-C6-haloalkoxycarbonyl group, a C3-C8-cycloalkoxycarbonyl group, a C3-C6-alkenyloxycarbonyl group, a benzyloxycarbonyl group, an optionally substituted benzyloxycarbonyl-C1-C6-alkyl group, or an optionally substituted phenoxycarbonyl-C1-C6-alkyl group;
R₂, R₅, and R₆ are the same or different from each other, and each represent a halogen atom, a C1-C6-alkyl group, a C1-C6-haloalkyl group, a C3-C8-cycloalkyl group, a benzyl group, a C3-C6-alkenyl group, a C3-C6-haloalkenyl group, a C3-C6-alkynyl group, a C3-C6-haloalkynyl group, a cyano-C1-C6-alkyl group, a C1-C4-alkoxy-C1-C4-alkyl group, a C1-C4-alkylthio-C1-C4-alkyl group, a carboxy-C1-C6-alkyl group, a (C1-C8-alkoxy)carbonyl-C1-C6-alkyl group, a (C1-C6-haloalkoxy)carbonyl-C1-C6-alkyl group, a hydroxy-C1-C2-alkyl group, a C1-C6-alkoxycarbonyl group, a C1-C6-haloalkoxycarbonyl group, a benzyloxycarbonyl group, or a C1-C6-alkylcarbonyl group, (provided that only R₂ may solely represents a hydroxyl group);
R₃ and R₄ are the same or different from each other, and each represent a hydrogen atom, a C1-C6-alkyl group, a C1-C6-haloalkyl group, a C3-C8-cycloalkyl group, a benzyl group, a C3-C6-alkenyl group, a C3-C6-haloalkenyl group, a C3-C6-alkynyl group, a C3-C6-haloalkynyl group, a cyano-C1-C6-alkyl group, a C1-C4-alkoxy-C1-C4-alkyl group, a C1-C4-alkylthio-C1-C4-alkyl group, a carboxy-C1-C6-alkyl group, a (C1-C8-alkoxy)carbonyl-C1-C6-alkyl group, a (C1-C6-haloalkoxy)carbonyl-C1-C6-alkyl group, a hydroxy-C1-C2-alkyl group, a C1-C6-alkoxycarbonyl group, a C1-C6-haloalkoxycarbonyl group, a benzyloxycarbonyl group, a C1-C6-alkylcarbonyl group, or a R₂₃R₂₄aminocarbonyl group ((R₂₃)R₂₄N-CO), wherein R₂₃ and R₂₄ are the same or different from each other, and each represent a hydrogen atom or a C1-C6-alkyl group, or R₂₃ and R₂₄ taken together represent trimethylene, tetramethylene, pentamethylene, ethyleneoxyethylene, ethyleneaminoethylene, or ethyleneoxyethylene;
R₇ and R₈ are the same or different from each other, and each represent a hydrogen atom, a C1-C6-alkyl group, or a C1-C6-haloalkyl group;
n1 represents 0 to 4;
n2 and n3 are the same or different from each other, and each represent 1 to 3;
provided that when n1 is 2 or above, then respective R₂ may be different from each other; when n2 is 2 or above, then respective R₅ may be different from each other; and when n3 is 2 or above, then respective R₆ may be different from each other.
and pharmaceutically acceptable salts thereof.

2. A triphenylmethane derivative according to claim 1 wherein, in the general formula [I], R₁ represents a group: -CR₁₁R₁₂CR₁₃R₁₄NR₁₅R₁₆ (wherein R₁₁ through R₁₆ are the same or different from each other, and each represent a hydrogen atom or a C1-C6-alkyl group, or R₁₅ and R₁₆ taken together represent trimethylene, tetramethylene, pentamethylene, ethyleneoxyethylene, ethyleneaminoethylene, or ethylenethioethylene;) R₂, R₅, and R₆ each represent a C1-C6-alkyl group, (provided that only R₂ may solely represent a hydroxyl group;)
and R₃ and R₄ are the same or different from each other, and each represent a hydrogen atom, a C1-C6-alkyl group, or a R₂₃R₂₄ aminocarbonyl group (wherein R₂₃ and R₂₄ are the same or different from each other, and each represent a hydrogen atom or a C1-C6-alkyl group, or R₂₃ and R₂₄ taken together represent trimethylene, tetramethylene, pentamethylene, ethyleneoxyethylene, ethyleneaminoethylene, or ethylenethioethylene.)

3. The triphenylmethane derivative according to claim 2, wherein R₁₁ through R₁₄ each represent a hydrogen atom; and R₁₅ and R₁₆ are the same or different from each other, and each represent a hydrogen atom or a C1-C6-alkyl group.

4. The pharmaceutical composition which comprise as an active ingredient a compound represented by the general formula [II]: 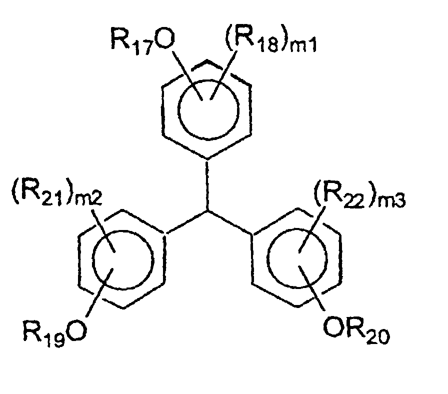 wherein,
[R₁₇, R₁₉, and R₂₀ are the same or different from each other, and each represent a hydrogen atom or an organic residue;
R₁₈, R₂₁, and R₂₂ are the same or different from each other, and each represent a halogen atom, a hydroxyl group, a C1-C6-alkyl group, a C1-C6-haloalkyl group, a C3-C8-cycloalkyl group, a benzyl group, a C3-C6-alkenyl group, a C3-C6-haloalkenyl group, a C3-C6-alkynyl group, a C3-C6-haloalkynyl group, a cyano-C1-C6-alkyl group, a C1-C4-alkoxy-C1-C4-alkyl group, a C1-C4-alkylthio-C1-C4-alkyl group, a carboxy-C1-C-6-alkyl group, a (C1-C8-alkoxy)carbonyl-C1-C6-alkyl group, a (C1-C6-haloalkoxy)carbonyl-C1-C6-alkyl group, a hydroxy-C1-C2-alkyl group, a C1-C6-alkoxycarbonyl group, a C1-C6-haloalkoxycarbonyl group, a benzyloxycarbonyl group, a C1-C6-alkylcarbonyl group, a C1-C6-alkoxy group, a benzyloxy group, a C1-C6-alkylcarbonyloxy group, or a benzoyloxy group;
m1 represents 0 to 4;
m2 and m3 are the same or different from each other, and each represent 1 to 4;
provided that when m1 is 2 or above, then respective R₁₈ may be different from each other; when m2 is 2 or above, then respective R₂₁ may be different from each other; and when m3 is 2 or above, then respective R₂₂ may be different from each other;]
or a pharmaceutically acceptable salt thereof.

5. The pharmaceutical composition according to claim 4, wherein, in the general formula [II], R₁₈, R₂₁, and R₂₂ each represent a hydrogen atom, a hydroxyl group, or a C1-C6-alkyl group;
R₁₇, R₁₉, and R₂₀ each represent a hydrogen atom, a C1-C6-alkyl group, a group: -CR₁₁R₁₂CR₁₃R₁₄NR₁₅R₁₆ (wherein R₁₁ through R₁₆ are the same or different from each other, and each represent a hydrogen atom or a C1-C6-alkyl group, or R₁₅ and R₁₆ taken together represent trimethylene, tetramethylene, pentamethylene, ethyleneoxyethylene, ethyleneaminoethylene, or ethylenethioethylene), or a R₂₃R₂₄ aminocarbonyl group (wherein R₂₃ and R₂₄ are the same or different from each other, and each represent a hydrogen atom or a C1-C6-alkyl group, or R₂₃ and R₂₄ taken together represent trimethylene, tetramethylene, pentamethylene, ethyleneoxyethylene, ethyleneaminoethylene, or ethylenethioethylene.)

6. The pharmaceutical composition according to claim 5, wherein R₁₁ through R₁₄ each represent a hydrogen atom, and R₁₅ and R₁₆ each represent a C1-C6-alkyl group.

7. Use of the compound represented by the general formula [II] in claim 4 as a pharmaceutical composition.
